# EUROPEAN PATENT SPECIFICATION

(11) **EP 2 903 602 B1**
(45) Date of publication and mention of the grant of the patent: **18.09.2019**
(21) Application number: 13844898.0
(22) Date of filing: 07.10.2013
(51) Int. Cl.: A61K 31/155, A61K 31/519, A61K 9/24, A61P 3/10, A61K 31/517

(54) **COMBINATION DRUG COMPRISING GEMIGLIPTIN AND METFORMIN, AND METHOD FOR THE PREPARATION THEREOF**
KOMBINATIONSPRÄPARAT MIT GEMIGLIPTIN UND METFORMIN SOWIE VERFAHREN ZUR HERSTELLUNG DAVON
ASSOCIATION MÉDICAMENTEUSE COMPRENANT DE LA GEMIGLIPTINE ET DE LA METFORMINE, ET SON PROCÉDÉ DE PRÉPARATION

(30) Priority: 08.10.2012 KR 20120111404
(43) Date of publication of application: 12.08.2015
(73) Proprietor: LG Chem, Ltd., Seoul 07336 (KR)
(72) Inventor: KIM, Geun Tae, Daejeon 305-738 (KR); YUN, Duck Il, Daejeon 305-738 (KR); PARK, Ki Sook, Daejeon 305-738 (KR)
(74) Representative: Hoffmann Eitle
(86) International application number: PCT/KR2013/008932
(87) International publication number: WO 2014/058188

(56) References cited:
- EP-A1- 2 356 985
- WO-A1-2012/049566
- WO-A1-2012/120040
- WO-A2-2012/090225
- US-A1- 2010 074 950
- US-A1- 2010 323 011
- US-A1- 2011 206 766
- RHEE E J ET AL: "Efficacy and safety of gemigliptin compared with sitagliptin added to ongoing metformin therapy in patients with type 2 diabetes inadequately controlled with metformin alone", DIABETOLOGIA, vol. 55, no. Suppl. 1, 24 August 2012 (2012-08-24), page S352, XP008178812, & 48TH ANNUAL MEETING OF THE EUROPEAN-ASSOCIATION-FOR-THE-STUDY-OF-DIAB ETES; BERLIN, GERMANY; OCTOBER 01 -05, 2012

## Description

### Technical Field

The present invention relates to a combination drug comprising gemigliptin and metformin as active components and method for the preparation thereof.

### Background Art

Diabetes is divided into two types-i.e., insulin-dependent type 1 diabetes and insulin-independent (insulin-resistant) type 2 diabetes which is found in 90% or more of diabetic patients.

Type 2 diabetes is a chronic and progressive disease derived from complex pathological physiology involving double endocrine actions of insulin-resistance and insulin-secretion disorder. The treatment of type 2 diabetes starts conventionally with diet and exercise, and leads to a pharmacotherapy alone in which oral antidiabetic drugs are used. Although in some patients blood glucose levels may be controlled by conventional pharmacotherapy alone in the early stage, in most patients a combination therapy is required several years after diagnosis since their blood glucose levels could not be controlled by pharmacotherapy alone. However, because type 2 diabetes is a progressive disease, most patients who have shown good initial responses to conventional combination therapy come to experience difficulty in maintaining stable blood glucose level for a long time and require an increased amount of administration or additional treatment. Although the currently used combination therapies have the possibility to increase blood glucose control, they have limitations of their own as well as adverse effects, such as hypoglycemia, weight gain, etc. Furthermore, the combination of two or more oral antidiabetics causes discomfort in many patients, and may result in patient reluctance to follow the medication regimen and thus treatment failure.

Gemigliptin, a newly developed DPP-IV inhibitor, activates incretin to show a stable decrease in blood glucose level. Furthermore, gemigliptin does not have defects of conventional oral antidiabetics such as hypoglycemia and weight gain, and shows low risk of cardiovascular disorders, so that it has already overtaken sulfonylureas.

Considering the above, the present inventors have conducted intensive researches on a combination of two or more antidiabetics having different mechanisms of action for enhancing the therapeutic effect, alleviating the adverse effects and improving patient compliance. As a result of such efforts, the present inventors have found that such purposes can be achieved by the combination of gemigliptin and metformin, and thus completed the present invention.

WO 2012/090225 discloses that metformin may be combined with a DPP-IV inhibitor which may be selected from inter alia gemigliptin. EP 2 356 985 discloses compositions of metformin and sitagliptin which are useful in treating diabetes. Rhee E J et al.: "Efficacy and safety of gemigliptin compared with sitagliptin added to ongoing metformin therapy in patients with type 2 diabetes inadequately controlled with metformin alone", Diabetologia, vol 55, supplement 1, 24 August 2012, page S352, discloses that gemigliptin is a safe add on therapy to metformin and gemigliptin with metformin performs comparably to metformin and sitagliptin.

### Disclosure of Invention

### Technical Problem

The object of the present invention is to provide a pharmaceutical composition for the prevention and treatment of diabetes which comprises as active components antidiabetics having different mechanisms of action.

Another object of the present invention is to provide a method for preparation of the pharmaceutical composition for the prevention and treatment of diabetes which comprises as active components antidiabetics having different mechanisms of action.

### Solution to Problem

Therefore, the present invention provides a pharmaceutical composition which consists of a first layer comprising metformin and a second layer comprising gemigliptin, wherein the first layer further comprises pregelatinized starch.

In the pharmaceutical composition according to the present invention, the first layer comprising metformin may be coated with the second layer comprising gemigliptin.

Another object of the present invention is accomplished by a method for the preparation of a combination drug comprising gemigliptin and metformin, which comprises the steps of:
(a) mixing gemigliptin with a pharmaceutically acceptable excipient;
(b) mixing metformin with a pharmaceutically acceptable excipient which is pregelatinized starch and granulating the mixture; and
(c) mixing the mixture prepared in step (a) with the granules prepared in step (b).

In the method according to the present invention, step (c) is preferably carried out by pressing the mixture comprising gemigliptin and the granules comprising metformin into a bilayer tablet. Alternatively, step (c) may be carried out by coating a tablet made by the granules comprising metformin with the mixture comprising gemigliptin.

According to the present invention, due to the combination of gemigliptin of a DPP-IV inhibitor and metformin as antidiabetics having different mechanisms of action, the therapeutic effect is enhanced by the synergistic action of the drugs, the adverse effects are alleviated, and patient compliance is improved.

Furthermore, the present invention provides a combination drug comprising gemigliptin and metformin in separate forms in order to overcome the problem caused by the combination thereof and to achieve the same dissolution as separate drugs.

Accordingly, the first aspect of the present invention relates to a pharmaceutical composition of a bilayer which consists of a first layer comprising metformin and a second layer comprising gemigliptin, wherein the first layer further comprises pregelatinized starch.

Another aspect of the present invention relates to a pharmaceutical composition in
which the first layer comprising metformin is coated with the second layer comprising gemigliptin.

In order to prepare the combination drug comprising gemigliptin and metformin in separate forms according to the present invention, gemigliptin is prepared as a mixture with an excipient, metformin is granulated, and then the combination drug of gemigliptin and metformin may be prepared by mixing the two.

Specifically, the method for the preparation of a combination drug according to the present invention comprises the steps of:
(a) mixing gemigliptin with a pharmaceutically acceptable excipient;
(b) wet-granulating a mixture of metformin and a pharmaceutically acceptable excipient, which is pregelatinized starch, and drying the granules; and
(c) mixing the mixture comprising gemigliptin prepared in step (a) with the granules comprising metformin prepared in step (b).

In one embodiment of the method according to the present invention, the mixture comprising gemigliptin and the granules comprising metformin are prepared separately, and then they are pressed into a bilayer tablet consisting of a gemigliptin layer and a metformin layer. The combination drug shows dissolution patterns of fast release of gemigliptin and sustained release of metformin, and the dissolution patterns do not affect each other.

In another embodiment of the present invention, a tablet comprising metformin is coated with a gemigliptin layer. The coated combination drug may be prepared by pressing the metformin granules into a tablet, which is further coated with gemigliptin mixed with coating base.

The pharmaceutical composition of the present invention can comprise a pharmaceutically acceptable carrier or additive. In one embodiment of the present invention, the pharmaceutical composition can comprise a pharmaceutically acceptable excipient, disintegrating agent, binder, lubricant, etc. The excipient may include, but is not limited to, microcrystalline cellulose. The disintegrating agent may include, but is not limited to, croscarmellose sodium, crospovidone, sodium starch glycolate, etc. The binder may include, but is not limited to, polyvinylpyrrolidone, copovidone, etc. The lubricant may include, but is not limited to, colloidal silicon dioxide, hydrous silicon dioxide, magnesium stearate, sodium stearyl fumarate (Pruv®), glyceryl behenate (Compritol 888®), calcium stearate, stearic acid, talc, etc.

In another embodiment of the present invention, the second layer comprising gemigliptin may not include a binder.

In addition, the pharmaceutical composition of the present invention may be film-coated. The agents available in the film-coating layer may include conventional ones, such as hydroxypropylmethyl cellulose, polyvinylpyrrolidone, copovidone, Opadry® series, Eudragit® series, but are not limited thereto.

In the combination drug according to the present invention, gemigliptin as an active component can be included in the form of gemigliptin tartrate • 1.5 hydrates and used in an amount of 10 to 80% by weight, preferably 10 to 70% by weight, based on the total weight of the gemigliptin layer. Metformin as another active component can be included in the form of metformin hydrochloride and used in an amount of 40 to 80% by weight, preferably 50 to 70% by weight, based on the total weight of the metformin layer.

In the present invention, the gemigliptin layer may comprise 20 to 90% by weight of a pharmaceutically acceptable additive, and the metformin layer may comprise 20 to 60% by weight of a pharmaceutically acceptable additive. Such a ratio applies to all the formulations of the present invention including the separated granules. If the formulation has a ratio out of the above range, it may create tablets that are too large and thus lower administration convenience. For administration convenience, the gemigliptin layer can be preferably 40% by weight or less, more preferably 30% by weight or less, most preferably 20% by weight or less, based on the metformin layer.

In the present invention, the metformin layer shows a dissolution pattern of sustained-release and may use various pharmaceutically acceptable components for the dissolution pattern. A pregelatinized starch is used to construct a sustained release matrix for this purpose. The sustained-release matrix component is included in an amount of 40 to 80% by weight, more preferably 50 to 70% by weight, most preferably 55 to 65% by weight, based on the weight of metformin hydrochloride.

For the pharmaceutical composition according to the present invention, a variety of pharmaceutically acceptable gemigliptin salts may be used, of which gemigliptin tartrate • 1.5 hydrates are the most preferred form. The structure and preparation method of gemigliptin tartrate • 1.5 hydrates are disclosed in KR Patent No. 0776623,. In addition, for the pharmaceutical composition according to the present invention, a variety of pharmaceutically acceptable metformin salts may be used, of which metformin hydrochloride is the most preferred form.

### Advantageous Effects of Invention

The pharmaceutical composition comprising gemigliptin and metformin according to the present invention has a superior effect on the prevention and treatment of diabetes and its complex diseases, and reduces adverse effects of each component due to the synergistic combination of the two components having different mechanisms of action. In addition, the present invention provides a combination drug comprising gemigliptin and metformin in separated forms so that the problems caused by the combination may be overcome, and the same dissolution rates of both components may be achieved compared with those of single-component drugs.

### Brief Description of Drawings

Figure 1 is a graph showing the results of a dissolution test of gemigliptin for the combination drugs of Examples 1 and 2 compared with the single-component drug of Comparative Example 1.
Figure 2 is a graph showing the results of a dissolution test of metformin for the combination drugs of Examples 1 and 2 compared with the single-component drug of Comparative Example 1.

### Mode for the Invention

The present invention is explained in more detail by the following examples. However, these examples seek to illustrate the present invention only.

### Examples 1 and 2: Preparation of bilayer tablets comprising gemigliptin and metformin

Combination drugs of gemigliptin and metformin in which the fast-release gemigliptin does not affect the sustained-release metformin were prepared in these Examples.

### Table 1

**[Table 1]**

| Component | Example 1 | Example 2 |
|---|---|---|
| Metformin layer | | |
| Metformin hydrochloride | 500.0 mg | 500.0 mg |
| Polyvinylpyrrolidone | 40.0 mg | 40.0 mg |
| Microcrystalline cellulose | 50.0 mg | 50.0 mg |
| Pregelatinized starch | 300.0 mg | 300.0 mg |
| Carbomer | 90.0 mg | 90.0 mg |
| Magnesium stearate | 20.0 mg | 20.0 mg |
| Sub-total | 1000.0 mg | 1000.0 mg |

| Gemigliptin layer | | |
|---|---|---|
| Gemigliptin tartrate· 1.5 hydrates | 68.9 mg | 34.45 mg |
| Microcrystalline cellulose | 121.1 mg | 155.55 mg |
| Croscarmellose sodium | 6.0 mg | 6.0 mg |
| Sodium stearyl fumarate | 4.0 mg | 4.0 mg |
| Sub-total | 200.0 mg | 200.0 mg |
| Total | 1200.0 mg | 1200.0 mg |

A mixture of metformin hydrochloride, microcrystalline cellulose and pregelatinized starch was granulated by using polyvinylpyrrolidone as a binder, and the prepared granules were dried and mixed with carbomer and magnesium stearate to prepare a metformin layer material. A simple mixture of all components including gemigliptin tartrate • 1.5 hydrates was prepared for the gemigliptin layer material. Both the metformin layer material and gemigliptin layer material were pressed into bilayer tablets, and the tablets were film-coated by using Opadry II.

### Comparative Example 1: Single-component drug of gemigliptin and single-component drug of metformin

Gemigliptin 50 mg tablet produced by LG Life Sciences Ltd. was used as a single-component drug of gemigliptin, and commercially available Glucophage XR 500 mg tablet was used as a single-component drug of metformin.

### Test Example 1: Dissolution test of gemigliptin and metformin for preparations according to the Examples and Comparative Example

For the bilayer tablets of Examples 1 and 2, and the single-component drugs of Comparative Example 1, a dissolution test was performed and compared under the following conditions.

### [Conditions for dissolution]

Eluate: pH 1.2 (900 mL)
Instrument: USP Basket Method, 100 rpm
Temperature: 37°C

### [Result]

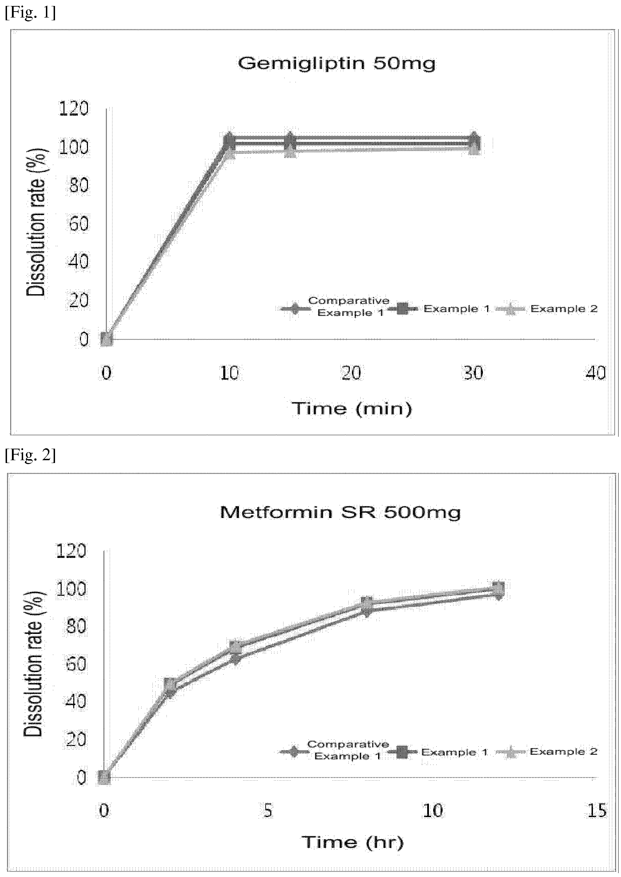

Figures 1 and 2 are graphs showing the results of a dissolution test of gemigliptin and metformin for the combination drugs of Examples 1 and 2 compared with the single-component drug of Comparative Example 1.

As is confirmed by Figures 1 and 2, the coated bilayer tablets comprising gemigliptin and metformin prepared in Examples 1 and 2 showed similar dissolution rates, compared with those of single-component drugs of Comparative Example 1.

## Claims

1. A combination drug which consists of a first layer comprising metformin and a second layer comprising gemigliptin,
wherein the first layer further comprises pregelatinized starch.

2. The combination drug according to claim 1, wherein the first layer comprising metformin is coated with the second layer comprising gemigliptin.

3. The combination drug according to claim 1, wherein the second layer further comprises microcrystalline cellulose.

4. A method for the preparation of the combination drug as defined in claim 1, which comprises the steps of:
(a) mixing gemigliptin with a pharmaceutically acceptable excipient;
(b) mixing metformin with a pharmaceutically acceptable excipient which is pregelatinized starch and granulating the mixture; and
(c) mixing the mixture prepared in step (a) with the granules prepared in step (b).

5. The method according to claim 4, wherein step (c) is carried out by pressing the mixture comprising gemigliptin prepared in step (a) and the granules comprising metformin prepared in step (b) into a bilayer tablet.

6. The method according to claim 4, wherein step (c) is carried out by coating a tablet made by the granules comprising metformin prepared in step (b) with the mixture comprising gemigliptin prepared in step (a).

7. The method according to claim 4, wherein the pharmaceutically acceptable excipient is selected from the group consisting of polyvinylpyrrolidone, microcrystalline cellulose, pregelatinized starch, carbomer, magnesium stearate, croscarmellose sodium and sodium stearyl fumarate.

## Patentansprüche

1. Kombinationspräparat, bestehend aus einer ersten Schicht, die Metformin umfasst, und einer zweiten Schicht, die Gemigliptin umfasst,
wobei die erste Schicht weiterhin vorgelatinierte Stärke umfasst.

2. Kombinationspräparat gemäß Anspruch 1, wobei die erste Schicht, die Metformin umfasst, mit der zweiten Schicht, die Gemigliptin umfasst, beschichtet ist.

3. Kombinationspräparat gemäß Anspruch 1, wobei die zweite Schicht weiterhin mikrokristalline Cellulose umfasst.

4. Verfahren zur Herstellung des Kombinationspräparats, wie in Anspruch 1 definiert, umfassend die Schritte:
(a) das Mischen von Gemigliptin mit einem pharmazeutisch verträglichen Hilfsstoff;
(b) das Mischen von Metformin mit einem pharmazeutisch verträglichen Hilfsstoff, der vorgelatinierte Stärke ist, und das Granulieren der Mischung; und
(c) das Mischen der in Schritt (a) hergestellten Mischung mit dem in Schritt (b) hergestellten Granulat.

5. Verfahren gemäß Anspruch 4, wobei Schritt (c) durchgeführt wird, indem die in Schritt (a) hergestellte Mischung, die Gemigliptin umfasst, und das in Schritt (b) hergestellte Granulat, das Metformin umfasst, zu einer Zweischichttablette gepresst werden.

6. Verfahren gemäß Anspruch 4, wobei Schritt (c) durchgeführt wird, indem eine Tablette aus dem in Schritt (b) hergestellten Granulat, das Metformin umfasst, mit der in Schritt (a) hergestellten Mischung, die Gemigliptin umfasst, beschichtet wird.

7. Verfahren gemäß Anspruch 4, wobei der pharmazeutisch verträgliche Hilfsstoff aus der Gruppe ausgewählt ist, die aus Polyvinylpyrrolidon, mikrokristalliner Cellulose, vorgelatinierter Stärke, Carbomer, Magnesiumstearat, Croscarmellose-Natrium und Natriumstearylfumarat besteht.

## Revendications

1. Association médicamenteuse qui est constituée par une première couche comprenant de la metformine et d'une seconde couche comprenant de la gémigliptine, dans laquelle la première couche comprend en outre de l'amidon prégélatinisé.

2. Association médicamenteuse selon la revendication 1, dans laquelle la première couche comprenant de la metformine est revêtue de la seconde couche comprenant de la gémigliptine.

3. Association médicamenteuse selon la revendication 1, dans laquelle la seconde couche comprend en outre de la cellulose microcristalline.

4. Procédé de préparation de l'association médicamenteuse selon la revendication 1, qui comprend les étapes consistant à :
(a) mélanger de la gémigliptine à un excipient pharmaceutiquement acceptable ;
(b) mélanger de la metformine à un excipient pharmaceutiquement acceptable qui est de l'amidon prégélatinisé et granuler le mélange ; et
(c) mélanger le mélange préparé à l'étape (a) aux granulés préparés à l'étape (b).

5. Procédé selon la revendication 4, dans lequel l'étape (c) est effectuée en pressant le mélange comprenant de la gémigliptine préparé à l'étape (a) et les granulés comprenant de la metformine préparés à l'étape (b) pour obtenir un comprimé bicouche.

6. Procédé selon la revendication 4, dans lequel l'étape (c) est effectuée en revêtant un comprimé constitué des granulés comprenant de la metformine préparés à l'étape (b) avec le mélange comprenant de la gémigliptine préparé à l'étape (a).

7. Procédé selon la revendication 4, dans lequel l'excipient pharmaceutiquement acceptable est sélectionné à partir du groupe constitué par de la polyvinylpyrrolidone, de la cellulose microcristalline, de l'amidon prégélatinisé, un carbomère, du stéarate de magnésium, de la croscarmellose sodique et du stéarylfumarate de sodium.
